# EUROPÄISCHE PATENTSCHRIFT

(11) **EP 4 368 247 B1**
(45) Veröffentlichungstag und Bekanntmachung des Hinweises auf die Patenterteilung: **11.03.2026**
(21) Anmeldenummer: 22206720.9
(22) Anmeldetag: 10.11.2022
(51) Int. Cl.: A61N 5/10, A61G 13/06, A61G 13/04

(54) **MEDIZINISCHE BEHANDLUNGSEINRICHTUNG MIT RINGGANTRY UND DREHBARER PATIENTENLIEGE**
MEDICAL TREATMENT DEVICE WITH RING GANTRY AND ROTATABLE PATIENT SUPPORT
DISPOSITIF DE TRAITEMENT MÉDICAL AVEC PORTIQUE ANNULAIRE ET SUPPORT DE PATIENT ROTATIF

(43) Veröffentlichungstag der Anmeldung: 15.05.2024
(73) Patentinhaber: Siemens Healthineers AG, 91301 Forchheim (DE)
(72) Erfinder: Neuber, Wolfgang, 92676 Eschenbach i. d. Opf. (DE)
(74) Vertreter: Siemens Healthineers Patent Attorneys

(56) Entgegenhaltungen:
- DE-B3- 102006 002 908
- US-A1- 2014 034 061
- US-A1- 2019 126 073
- US-A1- 2021 370 094
- US-A1- 2021 378 607

## Beschreibung

Die vorliegende Erfindung geht aus von einer medizinischen Behandlungseinrichtung, insbesondere einer Strahlentherapie-Einrichtung,
- wobei die medizinische Behandlungseinrichtung eine Ringgantry aufweist, die als stehender, um eine horizontale Längsrichtung umlaufender Ring ausgebildet ist und ein Isozentrum aufweist,
- wobei die medizinische Behandlungseinrichtung eine Patientenliege aufweist, die in der Längsrichtung gesehen vor der Ringgantry angeordnet ist,
- wobei die Patientenliege ein Basiselement, ein Zwischenelement, eine Lagerung und ein Liegenbrett aufweist,
- wobei das Basiselement an der Ringgantry befestigt ist, so dass es nicht oder ausschließlich in einer zur Längsrichtung orthogonalen horizontalen Querrichtung verlagerbar ist,
- wobei die Lagerung auf dem Zwischenelement angeordnet ist,
- wobei das Liegenbrett auf der Lagerung in einer zumindest im wesentlichen in der Längsrichtung verlaufenden horizontalen Schieberichtung verschiebbar ist.

Derartige Behandlungseinrichtungen sind allgemein bekannt. Insbesondere wird in diesem Zusammenhang auf das Dokument US 2021/0378607 A1 verwiesen.

Bei Anwendungen in der Strahlentherapie muss die Patientenliege oftmals um eine vertikale Hochachse gedreht werden, um Positionierungsfehler des Zielvolumens, das bestrahlt werden soll, auszugleichen. Das Zielvolumen ist in der Regel ein bestimmter Körperbereich eines Patienten. In manchen Fällen ist nur ein kleiner Drehwinkel möglich (beispielsweise von maximal 5°). In derartigen Fällen kann durch das Drehen der Patientenliege ausschließlich eine Korrektur von Positionierungsfehlern erfolgen. In anderen Fällen können auch größere Drehwinkel eingestellt werden (beispielsweise von bis zu 30°). In derartigen Fällen kann durch das Drehen auch ein Bestrahlen des Zielvolumens aus (bezüglich des Zielvolumens) deutlich unterschiedlichen Winkeln erfolgen, so dass das das Zielvolumen umgebende Volumen geringer belastet wird.

Optimal ist ein Drehen um eine durch das Isozentrum der Ringgantry gehende Hochachse. Denn dann erfolgt durch das Drehen keine translatorische Verlagerung des Zielvolumens.

Theoretisch ist es denkbar, eine entsprechende Positionierung der Hochachse dadurch zu erreichen, dass beidseits der Ringgantry jeweils ein Teil einer Tragstruktur auf kreisförmig gebogenen Führungselementen umläuft, wobei die beiden Teile der Tragstruktur mittels der beiden Führungselemente um eine gemeinsame Drehachse - nämlich die Hochachse - drehbar sind. Auf der Tragstruktur müsste in diesem Fall die Lagerung angeordnet sein, auf der das Liegenbrett linear verschiebbar ist. Diese prinzipiell mögliche Lösung ist jedoch mit erheblichen konstruktiven Nachteilen verbunden und wird daher in der Regel nicht ergriffen.

In der Praxis befinden sich Führungsstrukturen zum Ermöglichen einer Drehung vielmehr nur auf einer Seite der Ringgantry, nämlich auf derjenigen Seite, auf der sich die Patientenliege befindet.

Ein wichtiges Kriterium für die Auslegung der Patientenliege ist unter anderem die Stabilität, um eine reproduzierbare Positionierung des Patienten bzw. des Zielvolumens des Patienten erreichen zu können. Je größer die Steifigkeit der Patientenliege ist, desto genauer kann positioniert werden.

Es ist bekannt, das Zwischenelement über ein Drehlager auf dem Basiselement drehbar anzuordnen. Bei dieser Ausgestaltung treten jedoch zwei sich gegenseitig widersprechende Anforderungen auf. Denn einerseits sollte das Drehlager einen möglichst großen Durchmesser aufweisen, da die Stabilität des Drehlagers mit dem Durchmesser ansteigt. Andererseits sollte das Drehlager einen möglichst kleinen Durchmesser aufweisen, da der Ort der Hochachse in der Horizontalebene durch das Drehlager festgelegt ist und aufgrund des Durchmessers des Drehlagers umso weiter von dem Isozentrum entfernt ist, je größer der Durchmesser des Drehlagers ist. Je weiter aber die Hochachse vom Isozentrum entfernt ist, desto größer ist auch die bei einem Drehen auftretende translatorische Verlagerung des Zielvolumens.

In der Praxis wird der Durchmesser des Drehlagers meist relativ groß gewählt, um die nötige Stabilität zu gewährleisten. Der damit verbundene Nachteil der relativ großen translatorischen Verlagerung des Zielvolumens wird in Kauf genommen. Ein weiterer Nachteil der in der Praxis ergriffenen Lösung besteht darin, dass derartige Drehlager kostenintensiv sind.

Die Aufgabe der vorliegenden Erfindung besteht darin, Möglichkeiten zu schaffen, mittels derer auf einfache, kostengünstige und zuverlässige Art und Weise ein Drehen des Zwischenelements um eine vertikale Hochachse realisiert werden kann, wobei die Hochachse möglichst nahe an das Isozentrum der Ringgantry herangeführt werden kann, nach Möglichkeit sogar in das Isozentrum gelegt werden kann.

Die Aufgabe wird durch eine medizinische Behandlungseinrichtung mit den Merkmalen des Anspruchs 1 gelöst. Vorteilhafte Ausgestaltungen der medizinischen Behandlungseinrichtung sind Gegenstand der abhängigen Ansprüche 2 bis 10.

Erfindungsgemäß wird ein Betriebsverfahren der eingangs genannten Art dadurch ausgestaltet,
- dass das Zwischenelement mittels eines proximalen und eines distalen Lagerelements am Basiselement gelagert ist,
- dass beide Lagerelemente außerhalb eines von der Ringgantry umschlossenen Volumens und von der Ringgantry aus gesehen auf der gleichen Seite der Ringgantry angeordnet sind,
- dass das proximale Lagerelement näher an der Ringgantry angeordnet ist als das distale Lagerelement,
- dass das Zwischenelement mittels mindestens eines der beiden Lagerelemente derart geführt wird, dass es sich um eine vertikale Hochachse dreht, und
- dass die Hochachse bei dem proximalen Führungselement oder vom distalen Führungselement aus gesehen jenseits des proximalen Führungselements liegt, insbesondere im Isozentrum der Ringgantry.

Durch diese Ausgestaltung kann einerseits eine einfache Konstruktion erreicht werden, kann andererseits ein zuverlässiges Führen des Zwischenelements erreicht werden und kann darüber hinaus die Hochachse zumindest in die Nähe des Isozentrums der Ringgantry gebracht werden.

Es ist möglich, dass das proximale Lagerelement als Führungselement ausgebildet ist, welches das Zwischenelement auf einem inneren Kreisbogen mit einem inneren Bogenradius um die Hochachse führt, und das distale Lagerelement als Führungselement ausgebildet ist, welches das Zwischenelement auf einem äußeren Kreisbogen mit einem äußeren Bogenradius um die Hochachse führt. Diese Ausgestaltung weist den Vorteil auf, dass die Hochachse bis ins Isozentrum der Ringgantry gebracht werden kann.

Alternativ ist es möglich, dass das proximale Lagerelement als Drehlager ausgebildet ist, durch dessen Mittelpunkt die Hochachse geht. Diese Ausgestaltung weist den Vorteil auf, dass das eigentliche Führen des Zwischenelements durch das proximale Lagerelement alleine bewirkt wird. Das distale Führungselement übernimmt im wesentlichen nur noch die Aufgabe einer Abstützung.

Wiederum alternativ ist es möglich, dass das proximale Lagerelement als punktuelles Befestigungselement ausgebildet ist, durch das die Hochachse geht und an dem das Zwischenelement drehbar befestigt ist. In diesem Fall liegt die Hochachse direkt im Bereich des proximalen Lagerelements und damit außerhalb des von der Ringgantry umschlossenen Volumens. Das proximale Lagerelement ist in diesem Fall jedoch sehr einfach und auch sehr klein, so dass die Hochachse bis an den Rand der Ringgantry herangeführt werden kann.

Für den internen Aufbau des Zwischenelements sind verschiedene Ausgestaltungen möglich. Beispielsweise kann das Zwischenelement eine teleskopisch Hubsäule aufweisen, mittels derer die Lagerung in Vertikalrichtung angehoben und abgesenkt werden kann. Weiterhin ist eine Ausgestaltung nach Art eines Kniegelenks möglich. Auch andere Ausgestaltungen sind möglich. In einer bevorzugten Ausgestaltung der Behandlungseinrichtung ist jedoch vorgesehen,
- dass das Zwischenelement zum Basiselement hin eine bodennahe Platte aufweist,
- dass die Lagerung des Liegenbretts über einen Scherenmechanismus des Zwischenelements mit der bodennahen Platte verbunden ist,
- dass der Scherenmechanismus zwei Scherenarme aufweist, die mittig gelenkig miteinander verbunden sind, so dass die beiden Scherenarme ein X bilden,
- dass ein unterer Endpunkt des einen Scherenarms an der bodennahen Platte fixiert ist und ein unterer Endpunkt des anderen Scherenarms in einer Führung der bodennahen Platte geführt ist, so dass ein Abstand des unteren Endpunkts des anderen Scherenarms von der Ringgantry und dadurch ein vertikaler Abstand der Lagerung des Liegenbretts von dem Basiselement einstellbar ist,
- dass der untere Endpunkt des an der bodennahen Platte fixierten Scherenarms näher an der Ringgantry angeordnet ist als der untere Endpunkt des in der Führung der bodennahen Platte geführten Scherenarms.

Diese Ausgestaltung ermöglicht auf einfache und zuverlässige Weise eine Höhenverstellung der Lagerung des Liegenbretts. Weiterhin ergibt sich durch diese Ausgestaltung eine günstige Kraftführung.

Vorzugsweise ist der untere Endpunkt des an der bodennahen Platte fixierten Scherenarms im Bereich des proximalen Lagerelements angeordnet. Dadurch kann insbesondere die Gewichtskraft auf einfache Art und Weise von dem unteren Endpunkt des an der bodennahen Platte fixierten Scherenarms auf die bodennahe Platte und von dort auf das Basiselement übertragen werden.

Weiterhin ist vorzugsweise der untere Endpunkt des in der Führung der bodennahen Platte geführten Scherenarms in dem Zustand, in dem der Abstand des unteren Endpunkts des in der Führung der bodennahen Platte geführten Scherenarms von der Ringgantry minimal ist, im Bereich des distalen Lagerelements angeordnet. Dadurch kann im üblichen Betrieb, wenn die Lagerung des Liegenbretts nach oben gefahren ist und die höchsten Belastungen auftreten, insbesondere die auftretende Zugkraft auf einfache Art und Weise von dem unteren Endpunkt des in der Führung der bodennahen Platte geführten Scherenarms auf die bodennahe Platte und von dort auf das Basiselement übertragen werden.

Zur Aufnahme von in Vertikalrichtung gerichteten Kräften, die von dem Zwischenelement auf das distale Lagerelement wirken, weist das distale Lagerelement vorzugsweise eine sich in der Horizontalebene erstreckende Fixierungsplatte auf, die mit an dem Zwischenelement angeordneten Gegenelementen zusammenwirkt. Dadurch ist auf einfache Art und Weise eine Übertragung der in diesem Bereich wirkenden Kräfte von dem Zwischenelement auf das distale Lagerelement möglich.

Vorzugsweise umfassen die Gegenelemente obere Gegenelemente und untere Gegenelemente, wobei die oberen Gegenelemente von oben wirkende Druckkräfte auf die Fixierungsplatte übertragen und die unteren Gegenelemente von unten wirkende Zugkräfte auf die Fixierungsplatte übertragen. Dadurch gestaltet sich die Übertragung der Kräfte besonders einfach. Die Gegenelemente können insbesondere als Rollen ausgebildet sein, die auf der Fixierungsplatte abrollen.

Vorzugsweise sind die oberen Gegenelemente und/oder die unteren Gegenelemente am Zwischenelement in Vertikalrichtung justierbar angeordnet.

Wenn nur die oberen oder nur die unteren Gegenelemente justierbar sind, kann durch das entsprechende Justieren ein Spiel beseitigt werden, so dass sowohl die oberen als auch die unteren Gegenelemente passgenau an der Fixierungsplatte anliegen (und gegebenenfalls dort abrollen). Vorzugsweise können jedoch sowohl die oberen als auch die unteren Gegenelemente justiert werden, und zwar unabhängig voneinander. Dadurch kann nicht nur - wie zuvor - ein Spiel beseitigt werden, sondern darüber hinaus auch eine Justierung des Zwischenelements vorgenommen werden, insbesondere derart, dass die Schieberichtung exakt horizontal ist.

Aufgrund des Umstands, dass insbesondere ein Kippmoment um den unteren Endpunkt des an der bodennahen Platte fixierten Scherenarms über die Gegenelemente aufgefangen wird, kann das proximale Lagerelement derart (ergänze: klein) dimensioniert ist, dass es, wenn das distale Lagerelement nicht vorhanden wäre, durch die im Betrieb auftretenden Kräfte, insbesondere Scherkräfte bzw. Biegemomente zerstört würde. Damit kann das proximale Lagerelement sehr kostengünstig hergestellt werden.

Vorzugsweise ist ein Antrieb zum Drehen des Zwischenelements um die Hochachse als Omega-Antrieb ausgebildet. Dadurch kann insbesondere der Antrieb zum Drehen des Zwischenelements um die Hochachse in dem Basiselement angeordnet werden. Dies vereinfacht die Zugänglichkeit und auch die Energieversorgung dieses Antriebs.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die in Verbindung mit den Zeichnungen näher erläutert werden. Hierbei zeigen in schematischer Darstellung:
- FIG 1: eine medizinische Behandlungseinrichtung in einer perspektivischen Darstellung,
- FIG 2: ein Basiselement und eine bodennahe Platte eines Zwischenelements in einem unverdrehten Zustand,
- FIG 3: das Basiselement und die bodennahe Platte von FIG 2 in einem verdrehten Zustand,
- FIG 4: eine Patientenliege von der Seite,
- FIG 5: eine perspektivische Ansicht eines Basiselements und einer bodennahen Platte,
- FIG 6: ein Detail von FIG 5,
- FIG 7: ein Detail von FIG 6 von der Seite,
- FIG 8: einen Omega-Antrieb,
- FIG 9: eine Ringgantry und ein Basiselement und
- FIG 10: eine Ringgantry und ein weiteres Basiselement.

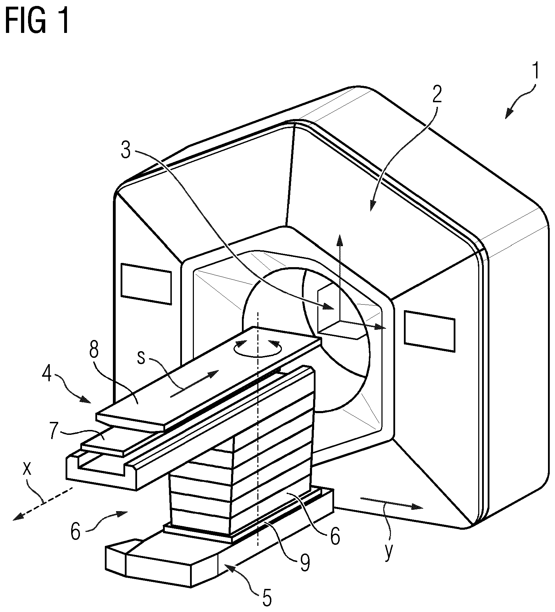

Gemäß den FIG 1 weist eine medizinische Behandlungseinrichtung 1 eine Ringgantry 2 auf. Die Ringgantry 2 ist ersichtlich als stehender, um eine horizontale Längsrichtung x umlaufender Ring ausgebildet. Die Ringgantry 2 weist ein Isozentrum 3 auf. Die Ringgantry 2 und damit die medizinische Behandlungseinrichtung 1 insgesamt können für eine Behandlung (Therapie) eines Patienten durch Bestrahlung bestimmt sein, also als Strahlentherapie-Einrichtung ausgebildet sein.

Die medizinische Behandlungseinrichtung 1 weist zusätzlich zur Ringgantry 2 eine Patientenliege 4 auf. Die Patientenliege 4 ist in der Längsrichtung x gesehen vor der Ringgantry 2 angeordnet. Die Patientenliege 4 weist ein Basiselement 5, ein Zwischenelement 6, eine Lagerung 7 und ein Liegenbrett 8 auf.

Das Basiselement 5 ist gemäß FIG 1 an der Ringgantry 2 befestigt. Es ist möglich, dass die Befestigung des Basiselements 5 starr ist, also unbeweglich und fix. Entsprechend der Darstellung in FIG 1 ist es jedoch in der Regel vorzuziehen, wenn das Basiselement 5 an der Ringgantry 2 derart befestigt ist, dass das Basiselement 5 in einer Querrichtung y verlagerbar ist. Die Querrichtung y verläuft ebenfalls horizontal, aber orthogonal zur Längsrichtung x. Eine weitergehende Verlagerbarkeit des Basiselements 2 ist in aller Regel nicht gegeben.

Die Lagerung 7 ist auf dem Zwischenelement 6 angeordnet. Auf der Lagerung 7 kann das Liegenbrett 8 in einer Schieberichtung s verschoben werden. Die Schieberichtung s verläuft horizontal, und zwar zumindest im wesentlichen in der Längsrichtung x. Auf dem Liegenbrett 8 kann ein Patient gelagert werden (in den FIG nicht dargestellt). Durch das Verschieben des Liegenbretts 8 kann ein Bereich des Patienten (sofern er auf dem Liegenbrett 8 gelagert ist) im Isozentrum 3 der Ringgantry 2 angeordnet werden. Die Schieberichtung s ist innerhalb der Horizontalebene variabel. Dies wird aus späteren Ausführungen noch ersichtlich werden.

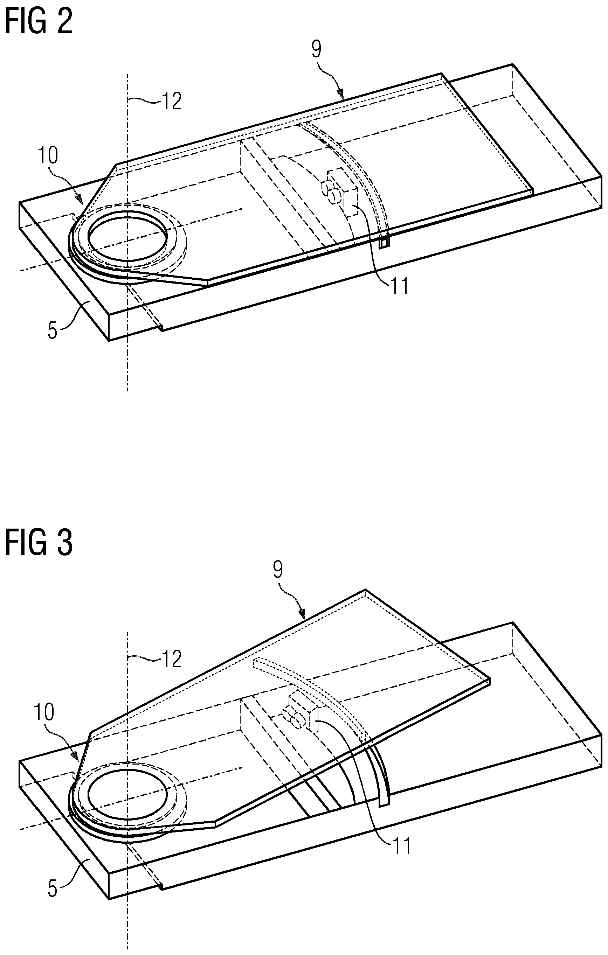

Die FIG 2 und 3 zeigen das Basiselement 5 und eine bodennahe Platte 9 des Zwischenelements 6.

Die bodennahe Platte 9 des Zwischenelements 6 ist gemäß den FIG 2 und 3 mittels zweier Lagerelemente 10, 11 am Basiselement 5 gelagert. Beide Lagerelemente 10, 11 sind außerhalb eines von der Ringgantry 2 umschlossenen Volumens angeordnet. Weiterhin sind beide Lagerelemente 10, 11 von der Ringgantry 2 aus gesehen und auf der gleichen Seite der Ringgantry 2 angeordnet. Das eine Lagerelement 10 ist näher an der Ringgantry 2 angeordnet als das andere Lagerelement 11. Das näher an der Ringgantry 2 angeordnete Lagerelement 10 wird nachstehend als proximales Element 10 bezeichnet, das weiter von der Ringgantry 2 entfernte Lagerelement 11 als distales Lagerelement 11.

Mittels mindestens eines der beiden Lagerelemente 10, 11 wird das Zwischenelement 6 derart geführt, dass es sich um eine vertikale Hochachse 12 dreht. Dies wird aus späteren Ausführungen noch ersichtlich werden. FIG 2 zeigt das Basiselement 5 und die bodennahe Platte 9 im unverdrehten Zustand der bodennahen Platte 9, FIG 3 im verdrehten Zustand der bodennahen Platte 9.

Die Hochachse 12 liegt in der Ausgestaltung der FIG 2 und 3 bei dem proximalen Führungselement 10. Beispielsweise ist in der Ausgestaltung gemäß FIG 2 das proximale Lagerelement 10 als Drehlager ausgebildet. In diesem Fall geht die Hochachse 12 durch den Mittelpunkt des Drehlagers. Bei anderen Ausgestaltungen kann die Hochachse 12 vom distalen Führungselement 11 aus gesehen auch jenseits des proximalen Führungselements 10 liegen, so dass also das proximale Führungselement 10 zwischen dem distalen Führungselement 11 und der Hochachse 12 liegt. Bei geeigneter Ausgestaltung kann die Hochachse 12 sogar im Isozentrum 3 der Ringgantry 2 liegen.

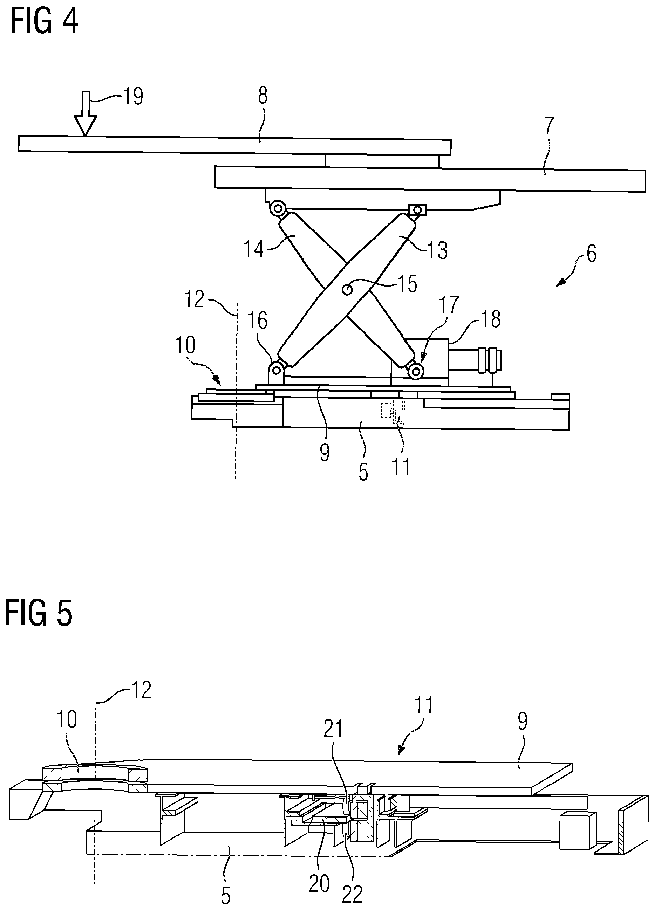

Das Zwischenelement 6 weist einen Mechanismus auf, mittels dessen die Lagerung 7 des Liegenbretts 8 in Vertikalrichtung verstellt werden kann. Gemäß Ausführungsbeispiel (siehe FIG 4) weist das Zwischenelement 6 zu diesem Zweck zusätzlich zur bodennahen Platte 9 einen Scherenmechanismus mit zwei Scherenarmen 13, 14 auf, wobei die beiden Scherenarme 13, 14 mittig gelenkig miteinander verbunden sind. Der Gelenkpunkt ist in FIG 4 mit dem Bezugszeichen 15 versehen. Durch die gelenkige Verbindung bilden die beiden Scherenarme 13, 14 ein X. Die bodennahe Platte 9 ist zum Basiselement 5 hin angeordnet. Der Scherenmechanismus ist oberhalb der bodennahen Platte 9 angeordnet.

Über den Scherenmechanismus ist die Lagerung 7 des Liegenbretts 8 mit der bodennahen Platte 9 verbunden. Konkret ist ein unterer Endpunkt 16 des einen Scherenarms 13 an der bodennahen Platte 9 fixiert. Ein unterer Endpunkt 17 des anderen Scherenarms 14 (in FIG 4 verdeckt) ist in einer Führung der bodennahen Platte 9 (in FIG 4 ebenfalls verdeckt) geführt. Das verdeckende Element ist ein Antrieb 18 für das entsprechende Verstellen dieses unteren Endpunkts 17. Durch entsprechendes Verstellen des Ortes dieses Endpunkts 17 kann somit ein Abstand dieses unteren Endpunkts 17 von der Ringgantry 2 eingestellt werden. Entsprechend der Einstellung dieses Abstands ist somit zugleich auch ein vertikaler Abstand der Lagerung 7 des Liegenbretts 8 von dem Basiselement 5 einstellbar. Entsprechend der Darstellung in FIG 4 ist der untere Endpunkt 16 des an der bodennahen Platte 9 fixierten Scherenarms 13 näher an der Ringgantry 2 angeordnet als der untere Endpunkt 17 des in der Führung der bodennahen Platte 9 geführten Scherenarms 14.

Vorzugsweise ist, wie auch aus FIG 4 ersichtlich, der untere Endpunkt 16 des an der bodennahen Platte 9 fixierten Scherenarms 13 im Bereich des proximalen Lagerelements 10 angeordnet. Weiterhin ist, wie ebenfalls aus FIG 4 ersichtlich, vorzugsweise der untere Endpunkt 17 des anderen Scherenarms 14 in dem Zustand, in dem der Abstand dieses unteren Endpunkts 17 von der Ringgantry 2 minimal ist (im Ergebnis also die Lagerung 7 und damit auch das Liegenbrett 8 so weit wie möglich angehoben sind), im Bereich des distalen Lagerelements 11 angeordnet.

Wenn der Patient im Isozentrum 3 der Ringgantry 2 positioniert ist, ist der Schwerpunkt des Liegenbretts 8 meist außerhalb des Bereichs angeordnet, in dem die Lagerung 7 mit dem Scherenmechanismus verbunden ist. Die im Schwerpunkt wirkende Gewichtskraft ist in FIG 4 durch einen Pfeil 19 angedeutet. Die Spitze des Pfeils 19 zeigt auf den Schwerpunkt. Aufgrund der Hebelwirkung können somit auf das distale Lagerelement 11 erhebliche Zugbelastungen wirken. Wenn umgekehrt das Liegenbrett 8 und damit auch der Patient nicht in das
Isozentrum 3 verschoben sind (beispielsweise weil der Patient sich gerade auf das Liegenbrett 8 legen will oder von ihm aufstehen will), befindet sich das Liegenbrett 8 - bezogen auf die Darstellung in FIG 4 - erheblich weiter rechts. In diesem Fall wirken erhebliche Druckbelastungen auf das distale Lagerelement 11. In beiden Fällen wirken die Kräfte somit in Vertikalrichtung.

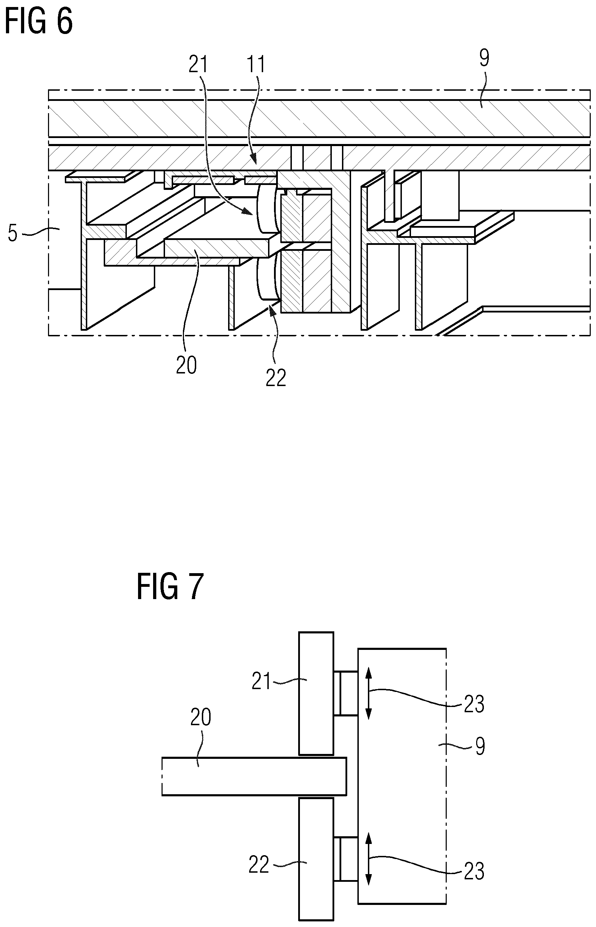

Zur Aufnahme derartiger in Vertikalrichtung gerichteter Kräfte, die von dem Zwischenelement 6 auf das distale Lagerelement 11 wirken, weist das distale Lagerelement 11 entsprechend der Darstellung in den FIG 5 und 6 eine Fixierungsplatte 20 auf. FIG 6 zeigt den in FIG 5 mit einem gestrichelten Kreis markierten Bereich. Die Fixierungsplatte 20 erstreckt sich in der Horizontalebene. Die Fixierungsplatte 20 wirkt mit Gegenelementen 21, 22 zusammen, die an dem Zwischenelement 6 (genauer: an der bodennahen Platte 9) angeordnet sind. Die Gegenelemente 21, 22 können insbesondere als Rollen ausgebildet sein, die auf der Fixierungsplatte 20 abrollen.

Die Gegenelemente 21 sind oberhalb der Fixierungsplatte 20 angeordnet und daher obere Gegenelemente. Die Gegenelemente 21 sind somit in der Lage, von oben wirkende Druckkräfte auf die Fixierungsplatte 20 zu übertragen. Umgekehrt sind die Gegenelemente 22 unterhalb der Fixierungsplatte 20 angeordnet und daher untere Gegenelemente. Die Gegenelemente 22 sind somit in der Lage, von unten wirkende Zugkräfte auf die Fixierungsplatte 20 zu übertragen.

Entsprechend der Darstellung in FIG 7 sind die oberen Gegenelemente 21 und/oder die unteren Gegenelemente 22 am Zwischenelement 6 (genauer: an der bodennahen Platte 9) in Vertikalrichtung justierbar angeordnet. Die Justierbarkeit ist in FIG 7 durch entsprechende Pfeile 23 angedeutet.

Aufgrund des Umstands, dass in einem nennenswerten Abstand voneinander zwei Lagerelemente 10, 11 vorhanden sind, kann das proximale Lagerelement 10 insbesondere derart dimensioniert sein, dass es durch die im Betrieb auftretenden Kräfte - insbesondere auftretende Scherkräfte bzw. Biegemomente - zerstört würde, wenn das distale Lagerelement 11 nicht vorhanden wäre.

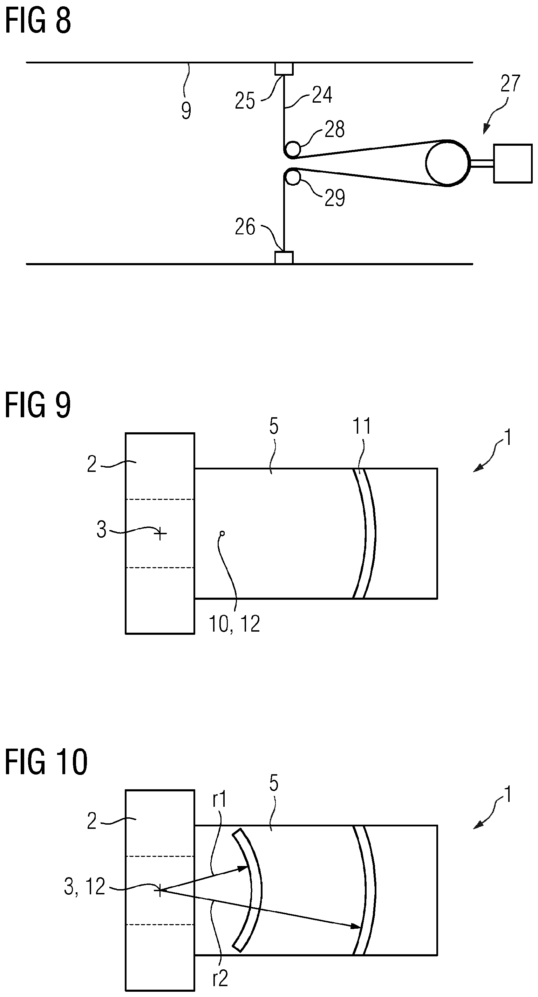

Das Drehen der bodennahen Platte 9 (und aller oberhalb der bodennahen Platte angeordneten Elemente der Patientenliege 4) um die Hochachse 12 erfolgt gemäß FIG 8 mittels eines sogenannten Omega-Antriebs. Bei einem Omega-Antrieb ist ein flexibles Kraftübertragungsmittel 24 vorhanden, beispielsweise ein Keilriemen, ein Zahnriemen oder eine Kette. Enden 25, 26 des Kraftübertragungsmittels 24 sind an dem zu bewegenden Element fixiert, hier an der bodennahen Platte 9. Das Kraftübertragungsmittel 24 wird einem eigentlichen Antrieb 27 über Umlenkrollen 28, 29 zugeführt. Sowohl der Antrieb 27 als auch die Umlenkrollen 28, 29 sind an demjenigen Element fixiert, gegenüber dem das zu bewegende Element bewegt werden soll. Vorliegend sind also der Antrieb 27 und die Umlenkrollen 28, 29 am Basiselement 5 angeordnet. Dreht der Antrieb 27 vorwärts, so verringert er die Länge an Kraftübertragungsmittel 24 zu dem einen Ende 25 um ein bestimmtes Ausmaß und vergrö-ßert die Länge an Kraftübertragungsmittel 24 zu dem anderen Ende 26 um das gleiche Ausmaß. Dreht der Antrieb 27 rückwärts, ist es umgekehrt. Da die Länge an Kraftübertragungsmittel 24 vom Antrieb 27 zu den Umlenkrollen 28, 29 konstant ist, müssen sich somit die Enden 25, 26 und mit ihnen die bodennahe Platte 9 um dieses Ausmaß bewegen.

Obenstehend wurde in Verbindung mit den FIG 2 bis 8 eine Ausgestaltung der vorliegenden Erfindung erläutert, bei welcher das proximale Lagerelement 10 als Drehlager ausgebildet ist und das distale Lagerelement 11 im wesentlichen nur vertikale Kräfte aufnehmen muss. Es sind jedoch auch andere Ausgestaltungen möglich.

So kann beispielsweise entsprechend der Darstellung in FIG 9 das proximale Lagerelement 10 als punktuelles Befestigungselement ausgebildet sein, an dem das Zwischenelement 6 drehbar befestigt ist. Beispielsweise kann das proximale Lagerelement 10 als Aufnahme für einen Bolzen der bodennahen Platte 9 ausgebildet sein. Auch die inverse Ausgestaltung ist möglich. In jedem Fall geht bei einer Ausgestaltung des proximalen Lagerelements 10 als punktuelles Befestigungselement die Hochachse 12 durch das proximale Lagerelement 10.

Weiterhin ist es möglich, dass das proximale Lagerelement 10 entsprechend der Darstellung in FIG 10 als Führungselement ausgebildet ist, welches das Zwischenelement 6 (bzw. die bodennahe Platte 9) auf einem inneren Kreisbogen mit einem inneren Bogenradius r1 um die Hochachse 12 führt. In diesem Fall ist das distale Lagerelement 11 entsprechend der Darstellung in FIG 10 als Führungselement ausgebildet, welches das Zwischenelement 6 (bzw. die bodennahe Platte 9) auf einem äußeren Kreisbogen mit einem äußeren Bogenradius r2 um die Hochachse führt. Die beiden Bogenradien r1, r2 sind derart aufeinander abgestimmt, dass sie beide um den selben Punkt geschwungen sind, also um die (gemeinsame) Hochachse 12. Insbesondere im Fall der Ausgestaltung gemäß FIG 10 kann die Hochachse 12 vom distalen Führungselement 11 aus gesehen jenseits des proximalen Führungselements 10 liegen, gegebenenfalls sogar im Isozentrum 3.

Zusammengefasst betrifft die vorliegende Erfindung somit folgenden Sachverhalt:
Eine medizinische Behandlungseinrichtung 1 weist eine Ringgantry 2 auf, die als stehender, um eine horizontale Längsrichtung x umlaufender Ring ausgebildet ist und ein Isozentrum 3 aufweist. Die medizinische Behandlungseinrichtung 1 weist weiterhin eine Patientenliege 4 auf, die in der Längsrichtung x gesehen vor der Ringgantry 2 angeordnet ist und ein Basiselement 5, ein Zwischenelement 6, eine Lagerung 7 und ein Liegenbrett 8 aufweist. Das Basiselement 5 ist an der Ringgantry 2 befestigt, so dass es nicht oder ausschließlich in einer zur Längsrichtung x orthogonalen horizontalen Querrichtung y verlagerbar ist. Die Lagerung 7 ist auf dem Zwischenelement 6 angeordnet, das Liegenbrett 8 auf der Lagerung 7 in einer zumindest im wesentlichen in der Längsrichtung x verlaufenden horizontalen Schieberichtung s verschiebbar. Das Zwischenelement 6 ist mittels eines proximalen und eines distalen Lagerelements 10, 11 am Basiselement 5 gelagert, wobei das proximale Lagerelement 10 näher an der Ringgantry 2 angeordnet ist als das distale Lagerelement 11. Beide Lagerelemente 10, 11 sind außerhalb eines von der Ringgantry 2 umschlossenen Volumens und von der Ringgantry 2 aus gesehen auf der gleichen Seite der Ringgantry 2 angeordnet. Das Zwischenelement 6 wird mittels mindestens eines der beiden Lagerelemente 10, 11 derart geführt, dass es sich um eine vertikale Hochachse 12 dreht. Die Hochachse 12 liegt bei dem proximalen Führungselement 10 oder vom distalen Führungselement 11 aus gesehen jenseits davon.

Die vorliegende Erfindung weist viele Vorteile auf. Zunächst ergibt sich eine mechanisch einfache und robuste, kostengünstige Realisierung der Drehbarkeit des Liegenbretts 8 um die Hochachse 12. Eine derartige Realisierung ist mit Standardkomponenten ("off the shelf") möglich. Falls die Hochachse 12 durch das Isozentrum 3 der Ringgantry 2 geht, kann direkt die - eigentlich gewünschte - Drehung um das Isozentrum 3 realisiert werden. In den anderen Fällen kann die Hochachse 12 nahe an das Isozentrum 3 der Ringgantry 3 gelegt werden. Dadurch ist der translatorische Offset, der sich beim Drehen des Zwischenelements 6 um die Hochachse 12 ergibt, klein. Sofern derartige Offsets durch translatorische Bewegungen kompensiert werden sollen, ist demzufolge auch das Ausmaß, zu dem derartige translatorischen Bewegungen erforderlich sind, entsprechend gering. Aufgrund des Abstands der beiden Lagerelemente 10, 11 voneinander kann eine hohe Stabilität der Patientenliege 4 und ihrer Komponenten erreicht werden.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizinische Behandlungseinrichtung, insbesondere Strahlentherapie-Einrichtung,
- wobei die medizinische Behandlungseinrichtung eine Ringgantry (2) aufweist, die als stehender, um eine horizontale Längsrichtung (x) umlaufender Ring ausgebildet ist und ein Isozentrum (3) aufweist,
- wobei die medizinische Behandlungseinrichtung eine Patientenliege (4) aufweist, die in der Längsrichtung (x) gesehen vor der Ringgantry (2) angeordnet ist,
- wobei die Patientenliege (4) ein Basiselement (5), ein Zwischenelement (6), eine Lagerung (7) und ein Liegenbrett (8) aufweist,
- wobei das Basiselement (5) an der Ringgantry (2) befestigt ist, so dass es nicht oder ausschließlich in einer zur Längsrichtung (x) orthogonalen horizontalen Querrichtung (y) verlagerbar ist,
- wobei die Lagerung (7) auf dem Zwischenelement (6) angeordnet ist,
- wobei das Liegenbrett (8) auf der Lagerung (7) in einer zumindest im wesentlichen in der Längsrichtung (x) verlaufenden horizontalen Schieberichtung (s) verschiebbar ist,
- wobei das Zwischenelement (6) mittels eines proximalen und eines distalen Lagerelements (10, 11) am Basiselement (5) gelagert ist,
- wobei beide Lagerelemente (10, 11) außerhalb eines von der Ringgantry (2) umschlossenen Volumens und von der Ringgantry (2) aus gesehen auf der gleichen Seite der Ringgantry (2) angeordnet sind,
- wobei das proximale Lagerelement (10) näher an der Ringgantry (2) angeordnet ist als das distale Lagerelement (11),
- wobei das Zwischenelement (6) mittels mindestens eines der beiden Lagerelemente (10, 11) derart geführt wird, dass es sich um eine vertikale Hochachse (12) dreht,
- wobei die Hochachse (12) bei dem proximalen Lagerelement (10) oder vom distalen Lagerelement (11) aus gesehen jenseits des proximalen Lagerelements (10) liegt, insbesondere im Isozentrum (3) der Ringgantry (2).

2. Behandlungseinrichtung nach Anspruch 1,
**dadurch gekennzeichnet,**
- **dass** das proximale Lagerelement (10) als Führungselement ausgebildet ist, welches das Zwischenelement (6) auf einem inneren Kreisbogen mit einem inneren Bogenradius (r1) um die Hochachse (12) führt, und das distale Lagerelement (11) als Führungselement ausgebildet ist, welches das Zwischenelement (6) auf einem äußeren Kreisbogen mit einem äußeren Bogenradius (r2) um die Hochachse (12) führt, oder
- **dass** das proximale Lagerelement (10) als Drehlager ausgebildet ist, durch dessen Mittelpunkt die Hochachse (12) geht, oder
- **dass** das proximale Lagerelement (10) als punktuelles Befestigungselement ausgebildet ist, durch das die Hochachse (12) geht und an dem das Zwischenelement (6) drehbar befestigt ist.

3. Behandlungseinrichtung nach Anspruch 1 oder 2,
**dadurch gekennzeichnet,**
- **dass** das Zwischenelement (6) zum Basiselement (5) hin eine bodennahe Platte (9) aufweist,
- **dass** die Lagerung (7) des Liegenbretts (8) über einen Scherenmechanismus des Zwischenelements (6) höhenverstellbar mit der bodennahen Platte (9) verbunden ist,
- **dass** der Scherenmechanismus zwei Scherenarme (13, 14) aufweist, die mittig gelenkig miteinander verbunden sind, so dass die beiden Scherenarme (13, 14) ein X bilden,
- **dass** ein unterer Endpunkt (16) des einen Scherenarms (13) an der bodennahen Platte (9) fixiert ist und ein unterer Endpunkt (17) des anderen Scherenarms (14) in einer Führung der bodennahen Platte (9) geführt ist, so dass ein Abstand des unteren Endpunkts (17) des anderen Scherenarms (14) von der Ringgantry (2) und dadurch ein vertikaler Abstand der Lagerung (7) des Liegenbretts (8) von dem Basiselement (5) einstellbar ist,
- **dass** der untere Endpunkt (16) des an der bodennahen Platte (9) fixierten Scherenarms (13) näher an der Ringgantry (2) angeordnet ist als der untere Endpunkt (17) des in der Führung der bodennahen Platte (9) geführten Scherenarms (14).

4. Behandlungseinrichtung nach Anspruch 3,
**dadurch gekennzeichnet,**
**dass** der untere Endpunkt (16) des an der bodennahen Platte (9) fixierten Scherenarms (13) im Bereich des proximalen Lagerelements (10) angeordnet ist.

5. Behandlungseinrichtung nach Anspruch 3 oder 4,
**dadurch gekennzeichnet,**
**dass** der untere Endpunkt (17) des in der Führung der bodennahen Platte (9) geführten Scherenarms (14) in dem Zustand, in dem der Abstand des unteren Endpunkts (17) des in der Führung der bodennahen Platte (9) geführten Scherenarms (14) von der Ringgantry (2) minimal ist, im Bereich des distalen Lagerelements (11) angeordnet ist.

6. Behandlungseinrichtung nach einem der obigen Ansprüche,
**dadurch gekennzeichnet,**
**dass** zur Aufnahme von in Vertikalrichtung gerichteten Kräften, die von dem Zwischenelement (6) auf das distale Lagerelement (11) wirken, das distale Lagerelement (11) eine sich in der Horizontalebene erstreckende Fixierungsplatte (20) aufweist, die mit an dem Zwischenelement (6) angeordneten Gegenelementen (21, 22) zusammenwirkt.

7. Behandlungseinrichtung nach Anspruch 6,
**dadurch gekennzeichnet,**
**dass** die Gegenelemente (21, 22) obere Gegenelemente (21) und untere Gegenelemente (22) umfassen, dass die oberen Gegenelemente (21) von oben wirkende Druckkräfte auf die Fixierungsplatte (20) übertragen und dass die unteren Gegenelemente (22) von unten wirkende Zugkräfte auf die Fixierungsplatte (20) übertragen.

8. Behandlungseinrichtung nach Anspruch 7,
**dadurch gekennzeichnet,**
**dass** die oberen Gegenelemente (21) und/oder die unteren Gegenelemente (22) am Zwischenelement (6) in Vertikalrichtung justierbar angeordnet sind.

9. Behandlungseinrichtung nach Anspruch 6, 7 oder 8,
**dadurch gekennzeichnet,**
**dass** das proximale Lagerelement (10) derart dimensioniert ist, dass es, wenn das distale Lagerelement (11) nicht vorhanden wäre, durch die im Betrieb auftretenden Kräfte, insbesondere Scherkräfte bzw. Biegemomente zerstört würde.

10. Behandlungseinrichtung nach einem der obigen Ansprüche,
**dadurch gekennzeichnet,**
**dass** ein Antrieb zum Drehen des Zwischenelements (6) um die Hochachse (12) als Omega-Antrieb ausgebildet ist.

## Claims

1. Medical treatment facility, in particular a radiotherapy facility,
- wherein the medical treatment facility has a ring gantry (2), which is embodied as an upright ring running around a horizontal longitudinal direction (x) and has an isocentre (3),
- wherein the medical treatment facility has a patient couch (4) that, viewed in the longitudinal direction (x), is arranged in front of the ring gantry (2),
- wherein the patient couch (4) has a base element (5), an intermediate element (6), a support (7) and a couch board (8),
- wherein the base element (5) is attached to the ring gantry (2), so that it is not able to be moved or is exclusively able to be moved in a horizontal transverse direction (y) orthogonal to the longitudinal direction (x),
- wherein the support (7) is arranged on the intermediate element (6),
- wherein the couch board (8) is able to be moved on the support (7) in a horizontal direction of movement (s) running at least essentially in the longitudinal direction (x),
- wherein the intermediate element (6) is supported by means of a proximal and a distal support element (10, 11) on the base element (5),
- wherein both support elements (10, 11) are arranged outside a volume surrounded by the ring gantry (2) and, viewed from the ring gantry (2), on the same side of the ring gantry (2),
- wherein the proximal support element (10) is arranged nearer to the ring gantry (2) than the distal support element (11),
- wherein the intermediate element (6) is guided by means of at least one of the two support elements (10, 11) in such a way that it rotates about a vertical axis (12),
- wherein the vertical axis (12) lies at the proximal support element (10) or, viewed from the distal support element (11) outwards, on the other side of the proximal support element (10), in particular in the isocentre (3) of the ring gantry (2).

2. Treatment facility according to claim 1,
**characterised in that**
- the proximal support element (10) is embodied as a guide element, which guides the intermediate element (6) on an inner circle arc with an inner arc radius (r1) about the vertical axis (12), and the distal support element (11) is embodied as a guide element, which guides the intermediate element (6) on an outer circle arc with an outer arc radius (r2) about the vertical axis (12), or
- the proximal support element (10) is embodied as a pivot bearing, through the centre point of which the vertical axis (12) passes, or
- the proximal support element (10) is embodied as a point-type attachment element, through which the vertical axis (12) passes and to which the intermediate element (6) is rotatably attached.

3. Treatment facility according to claim 1 or 2,
**characterised in that**
- the intermediate element (6) has a floor-level plate (9) towards the base element (5),
- the support (7) of the couch board (8) is connected height-adjustably via a scissor mechanism of the intermediate element (6) to the floor-level plate (9),
- the scissor mechanism has two scissor arms (13, 14), which are connected to one another by an articulated joint in the middle, so that the two scissor arms (13, 14) form an X,
- a lower end point (16) of the one scissor arm (13) is fixed to floor-level plate (9) and a lower end point (17) of the other scissor arm (14) is guided in a guide of the floor-level plate (9), so that a distance between the lower end point (17) of the other scissor arm (14) and the ring gantry (2) and thereby a vertical distance between the support (7) of the couch board (8) and the base element (5) is able to be set,
- the lower end point (16) of the scissor arm (13) fixed to the floor-level plate (9) is arranged nearer to the ring gantry (2) than the lower end point (17) of the scissor arm (14) guided in the guide of the floor-level plate (9).

4. Treatment facility according to claim 3,
**characterised in that**
the lower end point (16) of the scissor arm (13) fixed to the floor-level plate (9) is arranged in the area of the proximal support element (10).

5. Treatment facility according to claim 3 or 4,
**characterised in that**
the lower end point (17) of the scissor arm (14) guided in the guide of the floor-level plate (9) is arranged in the area of the distal support element (11) in the state in which the distance between the lower end point (17) of the scissor arm (14) guided in the guide of the floor-level plate (9) and the ring gantry (2) is minimal.

6. Treatment facility according to one of the above claims,
**characterised in that**
to accommodate forces directed in the vertical direction, which act from the intermediate element (6) on the distal support element (11), the distal support element (11) has a fixing plate (20) extending in the horizontal plane, which interacts with counter elements (21, 22) arranged on the intermediate element (6).

7. Treatment facility according to claim 6,
**characterised in that**
the counter elements (21, 22) comprise upper counter elements (21) and lower counter elements (22), the upper counter elements (21) transmit compressive forces acting from above to the fixing plate (20) and the lower counter elements (22) transmit tensile forces acting from below to the fixing plate (20).

8. Treatment facility according to claim 7,
**characterised in that**
the upper counter elements (21) and/or the lower counter elements (22) are arranged, adjustable in a vertical direction, on the intermediate element (6).

9. Treatment facility according to claim 6, 7 and 8,
**characterised in that**
the proximal support element (10) is dimensioned in such a way that, were the distal support element (11) not present, it would be destroyed by the forces occurring during operation, in particular shear forces or bending moments.

10. Treatment facility according to one of the above claims,
**characterised in that**
a drive for rotation of the intermediate element (6) about the vertical axis (12) is embodied as an Omega drive.

## Revendications

1. Dispositif de traitement médical, en particulier dispositif de radiothérapie,
- dans lequel le dispositif de traitement médical a un portique (2) annulaire, qui est constitué sous la forme d'un anneau debout faisant le tour autour d'une direction (x) longitudinale horizontale, et un isocentre (3),
- dans lequel le dispositif de traitement médical a une couchette (4) de patient, qui, considéré dans la direction (x) longitudinale, est disposée avant le portique (2) annulaire,
- dans lequel la couchette (4) de patient a un élément (5) de base, un élément (6) intermédiaire, une suspension (7) et une planche (8) de couchette,
- dans lequel l'élément (5) de base est fixé au portique (2) annulaire, de manière à ne pas pouvoir être déplacé ou à pouvoir être déplacé exclusivement dans une direction (y) transversale horizontale orthogonale à la direction (x) longitudinale,
- dans lequel la suspension (7) est disposée sur l'élément (6) intermédiaire,
- dans lequel la planche (8) de la couchette peut coulisser sur la suspension (7) dans une direction (s) horizontale de coulissement s'étendant au moins sensiblement dans la direction (x) longitudinale,
- dans lequel l'élément (6) intermédiaire est monté sur l'élément (5) de base au moyen d'un élément (10, 11) de palier proximal et distal,
- dans lequel les deux éléments (10, 11) de palier sont disposés à l'extérieur d'un volume enfermé par le portique (2) annulaire et, considéré depuis le portique (2) annulaire, du même côté du portique (2) annulaire,
- dans lequel l'élément (10) de palier proximal est disposé plus près du portique (2) annulaire que ne l'est l'élément (11) de palier distal,
- dans lequel l'élément (6) intermédiaire est, au moyen d'au moins l'un des deux éléments (10, 11) de palier, guidé de manière à tourner autour d'un axe (12) vertical,
- dans lequel l'axe (12) vertical dans l'élément (10) de palier proximal ou, considéré à partir de l'élément (11) de palier distal, est au-delà de l'élément (10) de palier proximal, en étant, en particulier à l'isocentre (3) du portique (2) annulaire.

2. Dispositif de traitement suivant la revendication 1, **caractérisé**
- **en ce que** l'élément (10) de palier proximal est constitué en élément de guidage, qui guide l'élément (6) intermédiaire sur un arc circulaire intérieur ayant un rayon (r1) intérieur de courbure autour de l'axe (12) vertical et l'élément (11) de palier distal est constitué sous la forme d'un élément de guidage, qui guide l'élément (6) intermédiaire sur un arc de cercle extérieur ayant un rayon (r2) de courbure extérieur autour de l'axe (12) vertical, ou
- **en ce que** l'élément (10) de palier proximal est constitué sous la forme d'un coussinet de pivotement au centre duquel passe l'axe (12) vertical, ou
- **en ce que** l'élément (10) de palier proximal est constitué sous la forme d'un élément de fixation ponctuel, dans lequel passe l'axe (12) vertical, et est fixé avec possibilité de tourner à l'élément (6) intermédiaire.

3. Dispositif de traitement suivant la revendication 1 ou 2, **caractérisé**
- **en ce que** l'élément (6) intermédiaire a, vers l'élément (5) de base, un plateau (9) proche du sol,
- **en ce que** la suspension (7) de la planche (8) de la couchette est, par un mécanisme à ciseau de l'élément (6) intermédiaire, reliée de manière réglable en hauteur au plateau (9) proche du sol,
- **en ce que** le mécanisme de ciseau a deux bras (13, 14) de ciseau, qui sont articulés entre eux au milieu, de manière à ce que les deux bras (13, 14) de ciseau forment un X,
- **en ce qu'**un point (16) inférieur d'extrémité de l'un des bras (13) de ciseau est fixé au plateau (9) proche du sol et un point (17) inférieur d'extrémité de l'autre bras (14) de ciseau est guidé dans un guidage du plateau (9) proche du sol, de manière à ce qu'une distance du point (17) inférieur d'extrémité de l'autre bras (14) de ciseau au portique (2) annulaire soit réglable et qu'ainsi une distance verticale de la suspension (7) de la planche (8) de la couchette à l'élément (5) de base soit réglable,
- **en ce que** le point (16) inférieur d'extrémité du bras (13) de ciseau fixé au plateau (9) proche du sol est disposé plus près du portique (2) annulaire que le point (17) inférieur d'extrémité du bras (14) de ciseau guidé dans le guidage du plateau (9) proche du sol.

4. Dispositif de traitement suivant la revendication 3, **caractérisé**
**en ce que** le point (16) inférieur d'extrémité du bras (13) de ciseau fixé au plateau (9) proche du sol est disposé dans la zone de l'élément (10) de palier proximal.

5. Dispositif de traitement suivant la revendication 3 ou 4, **caractérisé**
**en ce que** le point (17) inférieur d'extrémité du bras (14) de ciseau guidé dans le guidage du plateau (9) proche du sol est, dans l'état, dans lequel la distance du point (17) d'extrémité inférieur du bras (14) d'extrémité guidé dans le guidage du plateau (9) proche du sol au portique (2) d'entrée minimum, dans la zone de l'élément (11) de palier distal.

6. Dispositif de traitement suivant l'une des revendications précédentes,
**caractérisé**
**en ce que** pour l'absorption de forces dirigées dans la direction verticale, qui agissent de l'élément (6) intermédiaire sur l'élément (11) de palier distal, l'élément (11) de palier distal a un plateau (20) de fixation s'étendant dans le plan horizontal, qui coopère avec des éléments (21, 22) antagonistes disposés sur l'élément (6) intermédiaire.

7. Dispositif de traitement suivant la revendication 6, **caractérisé**
**en ce que** les éléments (21, 22) antagonistes comprennent des éléments (21) antagonistes supérieurs et des éléments (22) antagonistes inférieurs, **en ce que** les éléments (21) antagonistes supérieurs transmettent au plateau (20) de fixation des forces de pression agissant du haut et **en ce que** les éléments (22) antagonistes inférieurs transmettent au plateau (20) de fixation des forces de traction agissant d'en bas.

8. Dispositif de traitement suivant la revendication 7, **caractérisé**
**en ce que** les éléments (21) antagonistes supérieurs et/ou les éléments (22) antagonistes inférieurs sont montés sur l'élément (6) intermédiaire avec possibilité de réglage dans la direction verticale.

9. Dispositif de traitement suivant la revendication 6, 7 ou 8,
**caractérisé**
**en ce que** l'élément (10) de palier proximal est dimensionné de manière à ce que, si l'élément (11) de palier distal n'était pas présent, il serait détruit par les forces s'appliquant en fonctionnement, en particulier des forces de cisaillement ou des couples de flexion.

10. Dispositif de traitement suivant l'une des revendications précédentes,
**caractérisé**
**en ce qu'**un entraînement pour faire tourner l'élément (6) intermédiaire autour de l'axe (12) vertical est constitué sous la forme d'un entraînement en oméga.
